# EUROPEAN PATENT APPLICATION

(11) **EP 2 138 183 A1**
(43) Date of publication of application: **30.12.2009**
(21) Application number: 08722061.2
(22) Date of filing: 13.03.2008
(51) Int. Cl.: A61K 36/899, A23K 1/16, A23L 1/30, A23L 2/38, A23L 2/52, A61P 3/00, A61P 3/06

(54) **AMELIORATING OR PROPHYLACTIC AGENT FOR METABOLIC SYNDROME, AND BEVERAGE, FOOD AND FEED EACH COMPRISING THE SAME**

(30) Priority: 13.03.2007 JP 2007063901
(71) Applicant: Sapporo Breweries Limited, Tokyo 150-8522 (JP)
(72) Inventor: ARAKI, Shigeki, Tokyo 150-8522 (JP); KIHARA, Makoto, Tokyo 150-8522 (JP); SHIMIZU, Chikako, Tokyo 150-8522 (JP); HIRATA, Hiroshi, Tokyo 150-8522 (JP); IKEGAMI, Sachie, Tokyo 102-8357 (JP); AOE, Seiichiro, Tokyo 102-8357 (JP)
(74) Representative: Vossius & Partner
(86) International application number: PCT/JP2008/054657
(87) International publication number: WO 2008/111651

(57) **Abstract**

The present invention provides an ameliorative or preventive agent for metabolic syndrome comprising a barley bran or an α-amylase-treated product thereof as an active ingredient. The present invention also provides an ameliorative or preventive agent for metabolic syndrome comprising a water-insoluble component of an α-amylase-treated product of a barley bran as an active ingredient. These are naturally derived ameliorative or preventive agents for metabolic syndrome which can inhibit the accumulation of visceral fat to ameliorate or prevent metabolic syndrome.

## Description

### Technical Field

The present invention relates to an ameliorative or preventive agent for metabolic syndrome, as well as to beverages, foods and feeds each containing it.

### Background Art

Lifestyle-related diseases such as hypertension, hyperlipidemia and diabetes have been on the rise in recent years, and there has been increasing awareness of health. Such lifestyle-related diseases do not occur independently from each other, but rather are interrelated conditions caused by visceral fat obesity, which is characterized by accumulation of visceral fat. Recently, these lifestyle-related diseases have come to be collectively referred to as metabolic syndrome, and functional foods effective in the amelioration or prevention of metabolic syndrome are being actively developed.

In the initial stage of visceral fat obesity or metabolic syndrome, it is possible to prevent the onset and ameliorate the symptoms by altering dietary or lifestyle habits. As for these conditions, alimentary therapy and kinesitherapy are considered the basic treatments.

Alimentary therapy is mainly aimed at shifting the dietary menu to a low fat, low sucrose and high fiber diet to reduce energy intake, and forming regular dietary habits (Non-patent document 1). In kinesitherapy, the major aim is to increase consumption of ingested energy by performing aerobic exercise such as walking or swimming in combination with stretching, strength training or the like (Non-patent document 2).

Drug therapy is used when treatment by alimentary therapy or kinesitherapy is difficult. In drug therapy, a drug having a reducing effect on blood cholesterol or triglycerides is used, and as examples there may be mentioned absorption inhibitors (drugs that inhibit cellular absorption of lipid), synthesis inhibitors (drugs that inhibit synthesis of lipid by the liver) and excretion enhancers (drugs that enhance excretion of lipid by the liver) (Non-patent document 3).

Non-patent document 1: "Guidelines on dietary habits" [online], March 23, 2000, Ministry of Health, Labour and Welfare, "Press Releases" on web site of Ministry of Health, Labour and Welfare [search on November 21, 2006], internet <URL:http://www1.mhlwgo. jp/houdou/1203/h0323-1_a_11.html>
Non-patent document 2: "Guidelines on exercise for improved health 2006" [online], July 1, 2006, Ministry of Health, Labour and Welfare, "Health" on web site of Ministry of Health, Labour and Welfare [search on November 21, 2006], internet <URL:http://www. mhlw.go.jp/bunya/kenkou/undou.html>
Non-patent document 3: Itakura, "Selection and Proper Use of Hyperlipidemia Treatments", Japan Medical Publisher, 2000

### Disclosure of the Invention

### Problem to be Solved by the Invention

However, treatment of visceral fat obesity or metabolic syndrome by alimentary therapy or kinesitherapy requires making a treatment plan suited for the patient under the guidance of a doctor or dietician, and a long-term treatment plan is necessary to establish regular dietary habits and alter the body's constitution.

Also, in alimentary therapy, low calorie foods are used to reduce energy intake. However, virtually no foods that promote the breakdown and consumption of accumulated visceral fat are known. Consequently, if ingested in large amounts, even low fat, low sucrose and high fiber diets used in alimentary therapy cannot provide an ameliorative or preventive effect against metabolic syndrome.

Furthermore, although the drugs used in drug therapy, whose mechanisms of action have been known, can treat metabolic syndrome, their side effects are a concern.

It is an object of the present invention to provide a naturally derived ameliorative or preventive agent for metabolic syndrome which can inhibit the accumulation of visceral fat to ameliorate or prevent metabolic syndrome.

### Means for Solving the Problem

The present inventors found that when barley bran (outer layer of barley grain) is administered to disease models (KK mice) having accumulated visceral fat and exhibiting symptoms of metabolic syndrome, visceral fat accumulation and adipocyte hypertrophy are significantly inhibited compared to when milled barley grain (inner layer) is administered. The present invention has been completed based on this finding.

The present invention provides an ameliorative or preventive agent for metabolic syndrome comprising a barley bran or an α-amylase-treated product thereof as an active ingredient. The present invention also provides an ameliorative or preventive agent for metabolic syndrome comprising a water-insoluble component of an α-amylase-treated product of a barley bran as an active ingredient.

The ameliorative or preventive agent for metabolic syndrome according to the invention has a notably lower β-glucan content than milled barley grain (inner layer), and it is characterized by comprising, as an active ingredient, a barley bran, which is a by-product of barley grain milling, an α-amylase-treated product of a barley bran, or a water-insoluble component of an α-amylase-treated product of a barley bran.

Barley bran is a natural food material and is therefore far safer for the human body than chemically synthesized drugs. Its use makes it possible to treat or prevent metabolic syndrome at home to reduce the accumulation of visceral fat, even without the guidance of a doctor or dietician. Also, since most of the barley bran produced as a by-product when milling barley grain has traditionally been discarded, the use of a barley bran or a treated product thereof as an active ingredient of an ameliorative or preventive agent for metabolic syndrome allows more effective utilization of barley bran.

The ameliorative or preventive agent for metabolic syndrome according to the invention can inhibit the accumulation of visceral fat, thus reducing visceral fat and inhibiting adipocyte hypertrophy. Also, through such effects, it can ameliorate (treat or alleviate) or prevent visceral fat obesity and metabolic syndrome.

Metabolic syndrome is a condition primarily resulting from or consisting of visceral fat obesity, and visceral fat obesity is usually accompanied by hyperleptinemia and hyperinsulinemia. The ameliorative or preventive agent for metabolic syndrome according to the invention can also lower the blood (blood, serum or plasma) leptin and insulin levels, and through such effects, it can ameliorate (treat or alleviate) or prevent visceral fat obesity and metabolic syndrome.

When the ameliorative or preventive agent for metabolic syndrome according to the invention comprises a water-insoluble component of an α-amylase-treated product of a barley bran as an active ingredient, it typically contains a cell wall component as the water-insoluble component, and insoluble dietary fiber as the cell wall component. Also, the ameliorative or preventive agent for metabolic syndrome according to the invention usually further contains protein, lipid and ash. When it contains insoluble dietary fiber, protein, lipid and ash, the protein content is preferably 1 to 300 parts by weight, more preferably 30 to 240 parts by weight and even more preferably 50 to 200 parts by weight per 100 parts by weight of the insoluble dietary fiber, from the viewpoint of its more effective exertion of the aforementioned effects. From the same viewpoint, the lipid content is preferably 1 to 100 parts by weight, more preferably 5 to 80 parts by weight and even more preferably 10 to 60 parts by weight per 100 parts by weight of the insoluble dietary fiber. From the same viewpoint, the ash content is preferably 1 to 80 parts by weight, more preferably 5 to 70 parts by weight and even more preferably 10 to 60 parts by weight per 100 parts by weight of the insoluble dietary fiber. As examples of insoluble dietary fibers there may be mentioned hemicellulose, cellulose, lignin, pectin, insoluble β-glucan and the like.

In the present invention, in terms of obtaining greater visceral fat accumulation- and adipocyte hypertrophy-inhibiting effects, it is preferred that the barley bran be obtained by milling threshed barley grain to a milling degree of 5% to 30%.

Since the ameliorative or preventive agent for metabolic syndrome according to the invention has a visceral fat reducing effect and adipocyte hypertrophy inhibiting effect, it can be used as a visceral fat reducing agent or adipocyte hypertrophy inhibiting agent. Also, since the ameliorative or preventive agent for metabolic syndrome according to the invention can lower the leptin and insulin concentrations in the blood, it can be used as a blood leptin level lowering agent or blood insulin level lowering agent.

Furthermore, the ameliorative or preventive agent for metabolic syndrome according to the invention can be added to beverages, foods, feeds, etc. Beverages, foods, etc. containing the ameliorative or preventive agent for metabolic syndrome can be used in alimentary therapy for metabolic syndrome, because of their visceral fat reducing effect, adipocyte hypertrophy inhibiting effect, etc.

### Effects of the Invention

The ameliorative or preventive agent for metabolic syndrome according to the invention makes it possible to inhibit the accumulation of visceral fat in mammals, including humans, and to ameliorate (treat or alleviate) or prevent visceral fat obesity and metabolic syndrome.

### Brief Description of the Drawings

Fig. 1 is a graph showing the posterior abdominal wall fat weight of KK mice to which the barley bran fraction or wheat bran fraction is administered.
Fig. 2 is a graph showing the mesenteric fat weight of KK mice to which the barley bran fraction or wheat bran fraction is administered.
Fig. 3 is a graph showing the periepididymal fat weight of KK mice to which the barley bran fraction or wheat bran fraction is administered.
Fig. 4 is a graph showing the posterior abdominal wall adipocyte size of KK mice to which the barley bran fraction or wheat bran fraction is administered.
Fig. 5 is a graph showing the posterior abdominal wall fat weight of KK mice to which the outer layer fraction, defatted outer layer fraction, outer layer NDF fraction or germinated outer layer fraction is administered.
Fig. 6 is a graph showing the mesenteric fat weight of KK mice to which the outer layer fraction, defatted outer layer fraction, outer layer NDF fraction or germinated outer layer fraction is administered.
Fig. 7 is a graph showing the periepididymal fat weight of KK mice to which the outer layer fraction, defatted outer layer fraction, outer layer NDF fraction or germinated outer layer fraction is administered.
Fig. 8 is a graph showing the posterior abdominal wall adipocyte size of KK mice to which the outer layer fraction, defatted outer layer fraction, outer layer NDF fraction or germinated outer layer fraction is administered.
Fig. 9 is a graph showing the serum leptin level of KK mice to which the outer layer fraction, defatted outer layer fraction, outer layer NDF fraction or germinated outer layer fraction is administered.
Fig. 10 is a graph showing the serum insulin level of KK mice to which the outer layer fraction, defatted outer layer fraction, outer layer NDF fraction or germinated outer layer fraction is administered.

### Best Modes for Carrying Out the Invention

Preferred embodiments of the present invention will now be described in detail.

In one aspect, the ameliorative or preventive agent for metabolic syndrome according to the invention is characterized by comprising a barley bran or an α-amylase-treated product thereof as an active ingredient. In another aspect, it is characterized by comprising a water-insoluble component of an α-amylase-treated product of a barley bran as an active ingredient.

"Metabolic syndrome" as used herein refers to the condition defined by "Definition and the Diagnostic Standard for Metabolic Syndrome" (Nippon Naika Gakkai Zasshi (Journal of the Japanese Society of Internal Medicine), 94(4), 794-809, 2005) by the Committee to Evaluate Diagnostic Standards for Metabolic Syndrome.

Specifically, Japanese individuals are diagnosed as having metabolic syndrome, if the condition "waist circumference ≥ 85 cm for males or ≥ 90 cm for females (visceral fat area ≥ 100 cm² for both males and females)" and two or more of the following conditions (1) to (3) are met.
(1) Abnormal lipoprotein levels: High triglycerides (triglyceride level > 150 mg/dL) and/or low HDL cholesterol (HDL cholesterol level < 40 mg/dL)
(2) High blood pressure: Systolic pressure ≥ 130 mmHg and/or diastolic pressure ≥ 85 mmHg
(3) High blood sugar: Fasting glucose level ≥ 110 mg/dL

If an indivisual has been diagnosed as having metabolic syndrome based on these criteria, the indivisual has a lifestyle-related disease such as hypertension, hyperlipidemia or diabetes, and can be considered prone to serious diseases such as arteriosclerosis, myocardial infarction and cerebral infarction.

"Ameliorative or preventive agent for metabolic syndrome" as used herein means an agent that treats, alleviates or prevents metabolic syndrome.

The barley bran used in the invention can be obtained as a by-product when threshed barley grain is milled using a grain milling machine and the outer layer is removed. The milling treatment can also be performed using a rice polisher instead of a grain milling machine for barley, so long as it has an adjustable milling degree and can also be used for barley.

When milling is performed to obtain a processed cereal product, the cereal grain may be germinated to increase the content of an active ingredient such as GABA. The barley bran used in the invention may be germinated barley grain or nongerminated barley grain. When germinated barley grain is used, the germination period is preferably 12 hours to 10 days, more preferably 1 to 7 days and even more preferably 1 to 2 days in terms of obtaining a greater ameliorative or preventive effect against metabolic syndrome.

"α-Amylase-treated product of a barley bran" as used herein means a product obtained by mixing the barley bran with α-amylase to degrade the starch in the barley bran. The "α-amylase-treated product of a barley bran" may be a product from which the degraded starch has not been removed.

"Comprising as an active ingredient" means that the barley bran contains the ingredient in an amount sufficient to ameliorate (treat or alleviate) or prevent the symptoms of metabolic syndrome in a patient, or for example in an amount sufficient to exert a visceral fat reducing effect and/or adipocyte hypertrophy inhibiting effect.

When the ameliorative or preventive agent for metabolic syndrome according to the invention comprises a water-insoluble component of an α-amylase-treated product of a barley bran as an active ingredient, it typically contains a cell wall component as the water-insoluble component, and insoluble dietary fiber as the cell wall component. Also, the ameliorative or preventive agent for metabolic syndrome according to the invention usually further contains protein, lipid and ash. When it contains insoluble dietary fiber, protein, lipid and ash, the protein content is preferably 1 to 300 parts by weight, more preferably 30 to 240 parts by weight and even more preferably 50 to 200 parts by weight per 100 parts by weight of the insoluble dietary fiber, in terms of obtaining a greater ameliorative or preventive effect against metabolic syndrome. From the same viewpoint, the lipid content is preferably 1 to 100 parts by weight, more preferably 5 to 80 parts by weight and even more preferably 10 to 60 parts by weight per 100 parts by weight of the insoluble dietary fiber. From the same viewpoint, the ash content is preferably 1 to 80 parts by weight, more preferably 5 to 70 parts by weight and even more preferably 10 to 60 parts by weight per 100 parts by weight of the insoluble dietary fiber.

"Cell wall component" as used herein means a cell wall component in barley bran (outer layer of barley grain). For example, a component obtained by treating a water-insoluble component of an α-amylase-treated product of a barley bran with a neutral detergent fall under the "cell wall component". Also, "insoluble dietary fiber" means a dietary fiber (food component which cannot be digested by digestive enzymes in mammals) that is insoluble in water. As examples of insoluble dietary fibers there may be mentioned hemicellulose, cellulose, lignin, pectin, insoluble β-glucan and the like.

The barley bran is preferably obtained by milling threshed barley grain to a milling degree of 5% to 30%, and the milling degree is more preferably 5% to 25% and even more preferably 10% to 20%.

The milling degree is the numerical value (%) representing the degree of milling, and it is the value obtained by dividing the "weight of the outer layer removed from raw barley grain" by the "weight of the raw barley grain" and multiplying it by 100. Thus, "milling degree of 20%", for example, means that the percentage of outer layer removed as a barley bran is 20% and the percentage of inner layer of the milled barley grain is 80%. "Milling" and "milling treatment" mean removing bran layer (outer layer of barley grain).

The barley bran preferably contains vitamin E in an amount of 40 to 400 ppm by weight, and the vitamin E content is more preferably 100 to 400 ppm by weight and even more preferably 175 to 400 ppm by weight. The term "vitamin E" collectively refers to tocopherol and tocotrienol, and the vitamin E content is the sum of the tocopherol content and tocotrienol content.

As for vitamin E, tocotrienol is preferably contained in an amount of 3 0 to 3 00 ppm by weight, and the tocotrienol content is more preferably 100 to 300 ppm by weight and even more preferably 125 to 300 ppm by weight.

The ameliorative or preventive agent for metabolic syndrome according to the invention has a visceral fat reducing effect and/or adipocyte hypertrophy inhibiting effect.

"Visceral fat reducing effect" means the effect of inhibiting the accumulation of visceral fat or burning accumulated visceral fat, thus reducing the volume of visceral fat. As examples of visceral fat reducing effects there may be mentioned lipolytic effect, adipocyte proliferation inhibiting effect, inhibitory effects on the production of angiotensinogen, leptin, TNF-α, PAI-1, free fatty acids, etc., and stimulatory effect on the production of adiponectin.

"Adipocyte hypertrophy inhibiting effect" means the effect of inhibiting adipocyte hypertrophy resulting from excess accumulation of lipid in adipocytes. More specifically, it means the effect of inhibiting the increase in, or reducing, the size (long diameter) or volume of adipocytes.

As examples of animal models of metabolic syndrome, there may be mentioned: KK mice, which are characterized by overeating-induced obesity and accumulation of visceral fat; STR mice, which are a disease model of human gonarthrosis and exhibit accumulation of visceral fat; and Fischer 344 rats, which have increased cholesterol levels due to ingestion of a high fat diet. The presence or absence of a visceral fat accumulation inhibiting effect, visceral fat reducing effect or adipocyte hypertrophy inhibiting effect can be confirmed by administering barley bran to such an animal model, rearing it for a prescribed period, and then measuring organ weights and the size and volume of intraperitoneal adipocytes.

The ameliorative or preventive agent for metabolic syndrome according to the invention may be in the form of a solid (for example, powder obtained by freeze-drying), liquid (aqueous or nonaqueous solution or suspension), paste or the like. Also, it may be in the form of a powder, granule, tablet, capsule, liquid, suspension, emulsion, ointment, plaster or the like.

The aforementioned formulations can be prepared by mixing a barley bran, an α-amylase-treated product thereof or a water-insoluble component of an α-amylase-treated product of a barley bran with a pharmaceutically acceptable additive (excipient, binder, lubricant, disintegrant, emulsifier, surfactant, base, solubilizer, dispersant or the like).

As examples of excipients there may be mentioned lactose, sucrose, starch and dextrin. As examples of binders there may be mentioned polyvinyl alcohol, gum arabic, tragacanth, gelatin, hydroxypropyl methyl cellulose, hydroxy propyl cellulose, sodium carboxymethyl cellulose and polyvinylpyrrolidone. As examples of lubricants there may be mentioned magnesium stearate, calcium stearate and talc. As examples of disintegrants there may be mentioned crystalline cellulose, agar, gelatin, calcium carbonate, sodium hydrogen carbonate and dextrin. As examples of emulsifiers or surfactants there may be mentioned Tween 60, Tween 80, Span 80 and glycerol monostearate. As examples of bases there may be mentioned cetostearyl alcohol, lanolin, polyethylene glycol, rice bran oil, fish oil (DHA, EPA, etc.) and olive oil. As examples of solubilizers there may be mentioned polyethylene glycol, propylene glycol, sodium carbonate, sodium citrate and Tween 80. As examples of dispersants there may be mentioned the aforementioned surfactants, as well as polyvinyl alcohol, polyvinylpyrrolidone, methyl cellulose, hydroxy methyl cellulose and sodium alginate.

The ameliorative or preventive agent for metabolic syndrome according to the invention may be added to beverages, foods, feeds, etc. As examples of beverages to which it may be added, there may be mentioned water, soft drinks, fruit juices, milk beverages, alcoholic beverages and the like. As examples of foods to which it may be added, there may be mentioned grains such as rice and barley rice, and flours such as wheat flour. In particular, foods such as noodles, bread and confectioneries made from wheat flour are preferred. Processed foods made from wheat, barley or the like (for example, miso and shoyu) may also be mentioned. The ameliorative or preventive agent for metabolic syndrome according to the invention may also be used as a component of special health foods, special nutritious foods, nutritional supplements, health foods, functional foods, patient foods and the like. The content of an ameliorative or preventive agent for metabolic syndrome in a beverage or food, for example, may be any content that allows the agent to exert its beneficial effects, but it is preferably at least 5% by weight, more preferably at least 10% by weight, even more preferably at least 20% by weight and yet more preferably at least 30% by weight based on the total weight of the beverage or food.

The beverage, food, feed, etc. may further contain additives commonly used in the field. As examples of such additives there may be mentioned bittering agents, flavoring agents, apple fiber, soybean fiber, meat extract, black vinegar extract, gelatin, cornstarch, honey, animal or vegetable oils and fats; proteins such as gluten; beans such as soybean and pea; monosaccharides such as glucose; disaccharides such as sucrose, fructose and mannitol; polysaccharides such as dextrose and starch; sugar alcohols such as erythritol, xylitol and sorbitol; vitamins such as vitamin C; minerals such as zinc, copper and magnesium; and functional materials such as CoQ10, α-lipoic acid, carnitine and capsaicin. These additives may be used alone or in combinations of two or more.

### Examples

The present invention will now be explained in greater detail based on examples. However, the present invention is not limited to the examples below.

### [Example 1: Effects of barley bran on intraperitoneal fat accumulation and adipocyte hypertrophy]

### (Preparation of barley bran fraction and barley endosperm fraction)

180 g of threshed barley grain (variety: CDC Fibar) was milled for 2 minutes and 20 seconds to 2 minutes and 30 seconds using a grain milling machine (TM05C, Satake) with the milling degree set at 20%, and the removed outer layer of the barley grain was used as the barley bran fraction. Next, the barley grain that had been milled to a milling degree of 20% was milled for 2 minutes and 10 seconds to 2 minutes and 30 seconds with the milling degree set at 36%, and the inner layer of the barley grain further stripped of the outer layer was used as the barley endosperm fraction. Each of the fractions was subjected to gelatinization, and then freeze-dried and pulverized to produce powder.

The compositions of the fractions are shown in Table 1. Unless otherwise noted, the contents of the components are expressed in units of g/100g fraction in the table.

**[Table 1]**

| | Barley endosperm fraction | Barley bran fraction |
|---|---|---|
| Protein | 11.8 | 29.3 |
| Lipid (Crude fat) | 1.4 | 10.7 |
| Ash | 0.5 | 5.5 |
| Sugar | 67.6 | 24.0 |
| Water-soluble dietary fiber | 10.3 | 6.7 |
| (β-Glucan) | (9.2) | (5.7) |
| Insoluble dietary fiber | 2.7 | 21.1 |
| Total dietary fiber | 13.0 | 27.8 |
| Vitamin E (ppm) | 7 | 196 |
| (Tocotrienol (ppm)) | (3) | (142) |
| Energy (kcal) | 357 | 364 |

As shown in Table 1, a large difference was found in lipid content between the barley bran fraction and barley endosperm fraction. The lipid component was therefore further analyzed. As a result, the vitamin E content was only 7 ppm by weight in the barley endosperm fraction while it was 196 ppm by weight in the barley bran fraction. Also, the tocotrienol content was only 3 ppm by weight in the barley endosperm fraction while it was 142 ppm by weight in the barley bran fraction. These results demonstrate that the barley bran fraction used in this example was a fraction with a high content of vitamin E, especially tocotrienol.

### (Preparation of diets)

The diets to be fed to KK mice were prepared as follows, using powdered AIN93G diet as the base. As for the control group, powdered AIN93G diet was used as the diet to be fed to mice. As for the barley bran fraction-treated group and barley endosperm fraction-treated group, the diets to be fed to mice were prepared by blending the barley bran fraction or barley endosperm fraction with powdered AIN93G diet to a total dietary fiber content of 5%, and adding casein, cornstarch, soybean oil and cellulose so that the protein and lipid contents were equal in both groups.

The compositions of the diets are shown in Table 2. The contents of the components are expressed in units of g/kg diet in the table.

**[Table 2]**

| | Control group | Barley endosperm fraction-treated group | Barley bran fraction-treated group |
|---|---|---|---|
| Casein | 200 | 159 | 134 |
| L-Cystine | 3 | 3 | 3 |
| α-Cornstarch | 529 | 329 | 482 |
| Sucrose | 100 | 100 | 100 |
| Soybean oil | 70 | 66 | 50 |
| Cellulose | 50 | 12 | 0 |
| Whole grain barley | - | - | - |
| Barley endosperm fraction | - | 284 | - |
| Barley bran fraction | - | - | 183 |
| Mineral mixture | 35 | 35 | 35 |
| (AlN-93G composition) | | | |
| Vitamin mixture | 10 | 10 | 10 |
| (AlN-93 composition) | | | |
| Choline bitartrate | 2.5 | 2.5 | 2.5 |
| t-Butylhydroquinone | 0.014 | 0.014 | 0.014 |

### (Breeding of experimental animals)

Four-week-old male KK mice (CLEA Japan) were preliminarily bred for 1 week in a breeding room with a temperature of 22 ± 3°C (allowable range: 18 to 26°C), a relative humidity of 55 ± 20% (allowable range: 30 to 80%), a ventilation frequency of 12 times/hr and a light-dark cycle of 12 hours (illumination: 8:00 to 20:00). The general state of each mouse was observed each day during the preliminary feeding period, and the body weight was measured on the day following reception and on the final day of the period.

### (Feeding of diets)

After selecting 24 mice whose general states had been satisfactory during the preliminary feeding period, they were randomly divided into three groups (8 mice per group) so that the means and variances of body weights were roughly equal in the three groups. The mice in each group were allowed free access to the diet and tap water for 2 months.

Table 3 shows the initial body weight, final body weight, body weight gain, feed intake and feed efficiency for the KK mice in each group. The "initial body weight" is the weight of each mouse at the start of feeding, and the "final body weight" is the weight of each mouse at the end of feeding. The "body weight gain" is the body weight increase per day for each mouse during the feeding period. The "feed intake" is the dietary intake per day for each mouse during the feeding period, and the "feed efficiency" is the ratio of feed intake to body weight gain. The data in the table are expressed as mean ± standard deviation.

**[Table 3]**

| | Control group | Barley endosperm fraction-treated group | Barley bran fraction-treated group |
|---|---|---|---|
| Initial body weight (g) | 22.3 ± 1.3 | 22.2 ± 1.2 | 22.2 ± 1.3 |
| Final body weight (g) | 41.0 ± 1.5 | 41.6 ± 1.2 | 39.2 ± 2.5 |
| Body weight gain (g/day) | 0.31 ± 0.03 | 0.32 ± 0.02 | 0.28 ± 0.03 |
| Feed intake (g/day) | 4.0 ± 0.2 | 3.9 ± 0.3 | 3.7 ± 0.3 |
| Feed efficiency (%) | 7.7 ± 0.5 | 8.2 ± 0.4 | 7.5 ± 0.4 |

As shown in Table 3, there was no major variation in the aforementioned parameters between the groups, and even when the KK mice were fed a diet containing the barley bran fraction or barley endosperm fraction, the body weights etc. changed normally in the same manner as in the case of the control group. These results demonstrate that the barley bran and barley endosperm fractions have no toxicity that would affect the weight gain and feed intake for KK mice.

### (Measurement of fat weights and posterior abdominal wall adipocyte size, volume and number)

Upon completion of the two-month feeding period, the KK mice were dissected and their fat weights and posterior abdominal wall adipocyte size (long diameter), volume and number were measured (fasting for 4 hours before dissection).

Table 4 shows the weights of fat that had accumulated around different organs of the KK mice. Table 5 shows the posterior abdominal wall adipocyte size (long diameter), volume and number for the KK mice. The data in the table are expressed as mean ± standard deviation. The alphabet superscripts in the table represent the results of statistical analysis by the Turkey-Kramer method, and there are statistically significant differences (p < 0.05) between the data with different alphabet superscripts.

**[Table 4]**

| | Control group | Barley endosperm fraction-treated group | Barley bran fraction-treated group |
|---|---|---|---|
| Posterior abdominal wall fat weight (g) | 0.76 ± 0.07 ^{a} | 0.68 ± 0.09 ^{ab} | 0.63 ± 0.08^{b} |
| Periepididymal fat weight (g) | 1.41 ± 0.12 | 1.39 ± 0.09 | 1.45 ± 0.07 |

**[Table 5]**

| | Control group | Barley endosperm fraction-treated group | Barley bran fraction-treated group |
|---|---|---|---|
| Posterior abdominal wall adipocyte size (µm) | 94.4 ± 8.3 ^{a} | 94.2 ± 4.2 ^{a} | 86.6 ± 4.4 ^{b} |
| Posterior abdominal wall adipocyte volume (pL) | 474 ± 98 ^{a} | 440 ± 58 ^{a} | 343 ± 52 ^{b} |
| Posterior abdominal wall adipocyte number (×10⁶) | 1.76 ± 0.40 | 1.67 ± 0.35 | 1.97 ± 0.37 |

As shown in Table 4, the posterior abdominal wall fat weight was notably lower in the barley bran fraction-treated group than in the control group or barley endosperm fraction-treated group, and a statistically significant difference from the control group was found. On the other hand, no significant difference was found in periepididymal fat weight between the three groups. These results demonstrate that barley bran has a visceral fat reducing effect.

As shown in Table 5, the posterior abdominal wall adipocyte size and posterior abdominal wall adipocyte volume were notably smaller in the barley bran fraction-treated group than in the control group or barley endosperm fraction-treated group, and statistically significant differences from both groups were found. On the other hand, no significant difference was found in posterior abdominal wall adipocyte number between the three groups. These results demonstrate that barley bran has an adipocyte hypertrophy inhibiting effect.

### [Example 2: Comparison between effects of barley bran and wheat bran]

### (Preparation of barley bran fraction and wheat bran fraction)

180 g of threshed barley grain (variety: Ichibanboshi) was milled for approximately 1 minute using a grain milling machine (TM05C, Satake) with the milling degree set at 10%, and the removed outer layer of the barley grain was used as the barley bran fraction. Commercially available wheat bran (Wheat Fusuma, Nisshin Pharma) was used as the wheat bran fraction. Each of the fractions was subjected to gelatinization, and then freeze-dried and pulverized to produce powder.

### (Preparation of diets)

The diets to be fed to mice in the barley bran fraction-treated group and wheat bran fraction-treated group was prepared by blending the barley bran fraction or wheat bran fraction with powdered AIN93G diet to a total dietary fiber content of 5%, and adding casein, cornstarch, soybean oil and cellulose so that the protein and lipid contents were equal in both groups.

The compositions of the diets are shown in Table 6. The contents of the components are expressed in units of g/kg diet in the table.

**[Table 6]**

| | Control group | Wheat bran fraction-treated group | Barley bran fraction-treated group |
|---|---|---|---|
| Casein | 200 | 177 | 180 |
| L-Cystine | 3 | 3 | 3 |
| Cornstarch | 357 | 327 | 322 |
| α-Cornstarch | 132 | 132 | 132 |
| Sucrose | 100 | 100 | 100 |
| Soybean oil | 70 | 68 | 59 |
| Lard | 40 | 40 | 40 |
| Cellulose | 50 | - | - |
| Wheat bran fraction | - | 106 | - |
| Barley bran fraction | - | - | 116 |
| Mineral mixture | 35 | 35 | 35 |
| (AlN-93G composition) | | | |
| Vitamin mixture | 10 | 10 | 10 |
| (AlN-93 composition) | | | |
| Choline bitartrate | 2.5 | 2.5 | 2.5 |
| t-Sutyfhydroquinone | 0.014 | 0.014 | 0.014 |

### (Feeding of diets)

As in Example 1, four-week-old male KK mice (CLEA Japan) were preliminarily bred for 1 week, and mice whose general states had been satisfactory were randomly divided into three groups (8 mice per group) so that the means and variances of body weights were roughly equal in the three groups. The mice in each group were allowed free access to the diet and tap water for 2 months.

Table 7 shows the initial body weight, final body weight, body weight gain, feed intake and feed efficiency for the KK mice in each group. The "initial body weight", "final body weight", "body weight gain", "feed intake" and "feed efficiency" have the same meanings as above (in Table 3).

**[Table 7]**

| | Control group | Wheat bran fraction-treated group | Barley bran fraction-treated group |
|---|---|---|---|
| Initial body weight (g) | 24.7 ± 1.4 | 24.7 ± 1.5 | 24.7 ± 1.4 |
| Final body weight (g) | 40.1 ± 2.2 | 40.9 ± 1.8 | 40.3 ± 2.5 |
| Body weight gain (g/day) | 0.3 ± 0.05 | 0.3 ± 0.05 | 0.3 ± 0.05 |
| Feed intake (g/day) | 3.9 ± 0.3 | 4.0 ± 0.2 | 4.2 ± 0.5 |
| Feed efficiency (%) | 7.8 ± 1.2 | 7.9 ± 0.8 | 7.3 ± 0.9 |

As shown in Table 7, there was no major variation in the aforementioned parameters between the groups, and even when the KK mice were fed a diet containing the barley bran fraction or wheat bran fraction, the body weights etc. changed normally in the same manner as in the case of the control group. These results demonstrate that the barley bran and wheat bran fractions have no toxicity that would affect the weight gain and feed intake for KK mice.

### (Measurement of fat weights and posterior abdominal wall adipocyte size)

Upon completion of the two-month feeding period, the KK mice were dissected and their fat weights and posterior abdominal wall adipocyte size (long diameter) were measured (fasting for 4 hours before dissection).

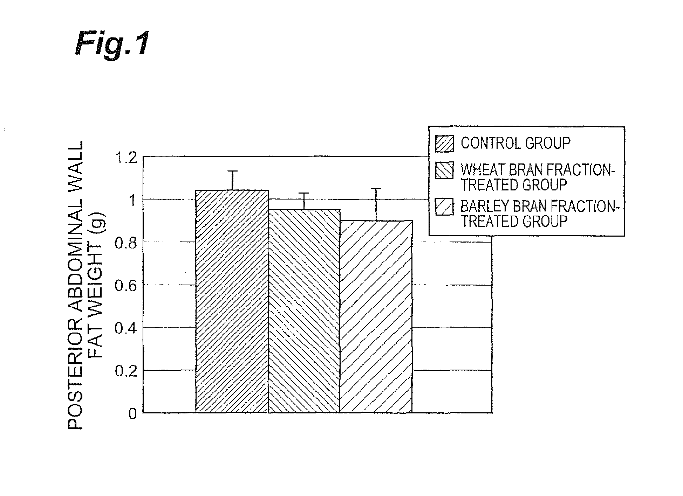

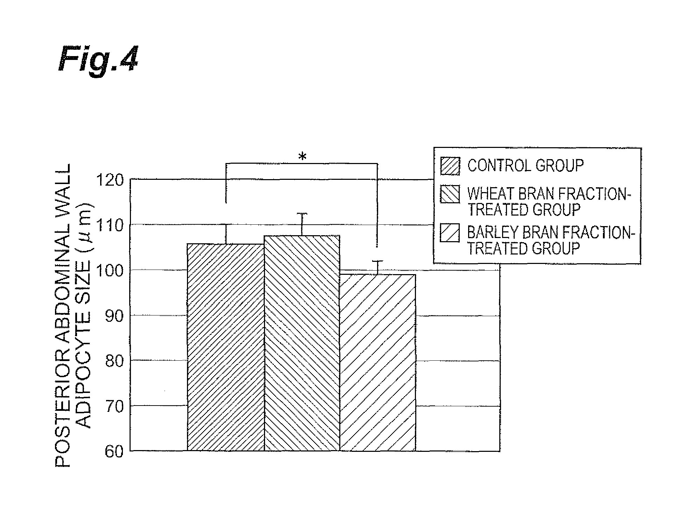

Figs. 1 to 3 are graphs showing the weights of fat that had accumulated around different organs of the KK mice after 2 months of ingestion of barley bran fraction or wheat bran fraction. Fig. 4 is a graph showing the posterior abdominal wall adipocyte size for the KK mice after 2 months of ingestion of barley bran fraction or wheat bran fraction. The data in the graphs are expressed as mean ± standard deviation. A single asterisk indicates a statistically significant difference (p < 0.05) between the two data.

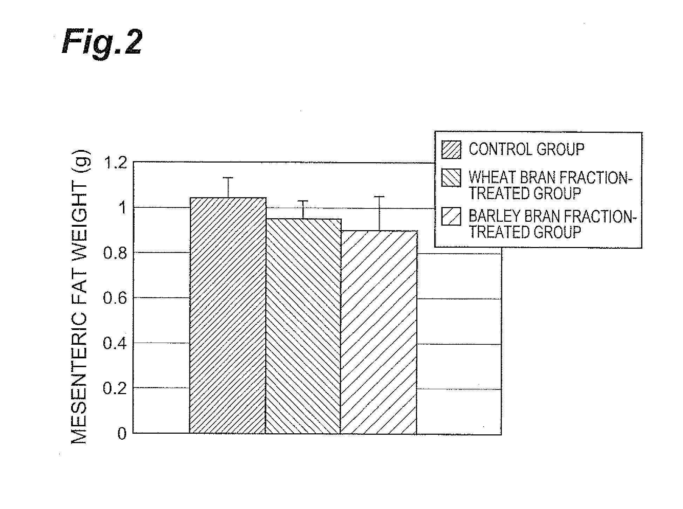

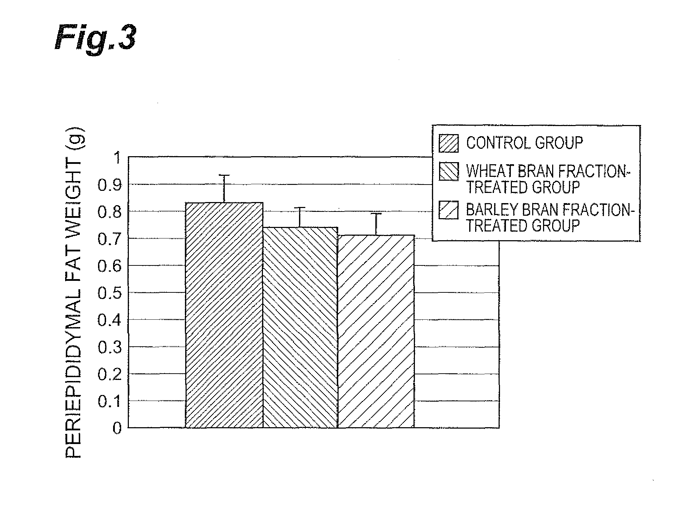

As shown in Figs. 1 to 3, the posterior abdominal wall fat weight (Fig. 1), mesenteric fat weight (Fig. 2) and periepididymal fat weight (Fig. 3) tended to decrease in both the barley bran fraction-treated group and wheat bran fraction-treated group compared to the control group, and the visceral fat reducing effect of the barley bran fraction was stronger than that of the wheat bran fraction.

As shown in Fig. 4, the posterior abdominal wall adipocyte size was notably smaller in the barley bran fraction-treated group than in the control group, and a statistically significant difference from the control group was found. On the other hand, in the wheat bran fraction-treated group, the posterior abdominal wall adipocyte size was larger than in the control group, and no adipocyte hypertrophy inhibiting effect was observed. These results demonstrate that barley bran has an adipocyte hypertrophy inhibiting effect, and that this effect is unique to barley bran and not seen with wheat bran.

Examples 1 and 2 demonstrate that barley bran exert a visceral fat reducing effect and adipocyte hypertrophy inhibiting effect, and is therefore useful as an ameliorative or preventive agent for metabolic syndrome.

### [Example 3 (Food example 1): Steamed bread]

Milk, egg and sugar were mixed and soft flour, barley bran and baking powder were then added in the proportions shown in Table 8 (Test foods 1 and 2) to prepare a dough. Next, the dough was cast into a prescribed mold and steamed for 15 minutes in a steamer to obtain a steamed bread.

**[Table 8]**

| | Test food 1 | Test food 2 |
|---|---|---|
| Soft flour | 70 g | 50 g |
| Barley bran | 30 g | 50 g |
| Balking powder | 6 g | 6 g |
| Milk | 50 g | 50 g |
| Egg | 75 g | 75 g |
| Sugar | 50 g | 50 g |
| Salad oil | 6 g | 6 g |

### [Example 4 (Food example 2): Leaf pie]

Hard flour, soft flour and barley bran were mixed in the proportions shown in Table 9 (Test foods 1 and 2) to prepare a dough. Next, granulated sugar was sprinkled over the dough folded in a prescribed shape and the dough was baked in an oven at 200°C for 15 minutes to obtain a leaf pie.

**[Table 9]**

| | Test food 1 | Test food 2 |
|---|---|---|
| Hard flour | 156 g | 37 g |
| Soft flour | 75 g | 18 g |
| Barley bran | 99 g | 55 g |
| Unsalted butter | 240 g | 80 g |
| Salt | Small amount | Small amount |
| Cold water | 195 g | 65 g |
| Granulated sugar | Moderate amount | Moderate amount |

### [Example 5 (Food example 3): Cookie]

Soft flour, barley bran and baking powder were mixed in the proportions shown in Table 10 (Test foods 1 to 3) to prepare a dough. Next, the dough was molded into a prescribed shape and baked in an oven at 150°C for 25 minutes to obtain a cookie.

**[Table 10]**

| | Test food 1 | Test food 2 | Test food 3 |
|---|---|---|---|
| Soft flour | 70 g | 60 g | 50 g |
| Barley bran | 30 g | 40 g | 50 g |
| Baking powder | 4 g | 4 g | 4 g |
| Margarine | 50 g | 50 g | 50 g |
| Sugar | 30 g | 30 g | 30 g |
| Egg | 60 g | 60 g | 60 g |
| Vanilla essence | Small amount | Small amount | Small amount |

### [Example 6: Effects of α-amylase-treated product of barley bran on intraperitoneal fat accumulation and adipocyte hypertrophy]

### (Preparation of outer layer fraction, defatted outer layer fraction, outer layer NDF fraction and germinated outer layer fraction)

180 g of threshed barley grain (variety: CDC Fibar) was milled for approximately 2 to 3 minutes using a grain milling machine (TM05C, Satake) with the milling degree set at 20%, and the removed outer layer of the barley grain was used as the outer layer (barley bran) fraction. Also, the extract obtained by extracting the outer layer of the barley grain with hexane was used as the defatted outer layer fraction. Also, the outer layer of the barley grain was treated with α-amylase, and further treated with a neutral detergent solution (a solution obtained by heating and dissolving 30 g of sodium lauryl sulfate, 18.61 g of EDTA·2Na, 6.81 g of sodium borate decahydrate, 4.56 g of anhydrous disodium phosphate and 10 mL of ethylene glycol monoethyl ether in 1 L of distilled water; pH: 6.9-7.1) to obtain a product which was used as the outer layer NDF fraction. Also, barley grain (variety: CDC Fibar) that had been subjected to germination for 2 days was treated in the same manner as in the case of the preparation of the outer layer fraction described above, to obtain the outer layer of the barley grain, which was used as the germinated outer layer fraction. Each of the fractions was subjected to gelatinization, and then freeze-dried and pulverized to produce powder.

The compositions of the fractions are shown in Table 11. The contents of the components are expressed in units of g/100g fraction in the table. In all of the fractions, β-glucan accounted for at least 80% by weight of the water-soluble dietary fiber.

**[Table 11]**

| | Outer layer fraction | Defatted outer layer fraction | Outer layer NDF fraction | Germinate outer layer fraction |
|---|---|---|---|---|
| Protein | 30.1 | 34.1 | 4.3 | 30.0 |
| Lipid (Crude fat) | 10.0 | 0.1 | 1.1 | 10.3 |
| Ash | 5.5 | 6.1 | 1.2 | 5.4 |
| Water-soluble dietary fiber | 6.5 | 7.9 | 1.2 | 6.5 |
| Insoluble dietary fiber | 21.3 | 25.0 | 92.5 | 22.7 |
| Total dietary fiber | 27.8 | 32.9 | 93.7 | 29.2 |

As shown in Table 11, the outer layer NDF fraction is composed mainly of cell wall components, particularly insoluble dietary fiber. The insoluble dietary fiber in the outer layer NDF fraction corresponds to what is commonly referred to as NDF (neutral detergent fiber) (for example, hemicellulose, cellulose, lignin, pectin or insoluble β-glucan).

### (Preparation of diets)

The diets to be fed to KK mice were prepared as follows, using powdered AIN93G diet as the base. As for the control group, powdered AIN93G diet was used as the diet to be fed to mice. As for the outer layer fraction-treated group, defatted outer layer fraction-treated group, outer layer NDF fraction-treated group and germinated outer layer fraction-treated group, the diets to be fed to mice were prepared by blending the outer layer fraction, defatted outer layer fraction, outer layer NDF fraction or germinated outer layer fraction with powdered AIN93G diet to a total dietary fiber content of 5%, and adding casein, cornstarch, soybean oil and cellulose so that the protein and lipid contents were equal in these groups.

The compositions of the diets are shown in Table 12. The contents of the components are expressed in units of g/kg diet in the table. Also, "control", "outer layer", "defatted outer layer", "outer layer NDF" and "germinated outer layer" respectively refer to the control group, outer layer fraction-treated group, defatted outer layer fraction-treated group, outer layer NDF fraction-treated group and germinated outer layer fraction-treated group (the same holds for the other tables and graphs).

**[Table 12]**

| | Control | Outer layer | Defatted outer layer | Outer layer NDF | Germinated outer layer |
|---|---|---|---|---|---|
| Casein | 200.0 | 134.9 | 141.8 | 198.3 | 137.5 |
| L-Cystine | 3 | 3 | 3 | 3 | 3 |
| α-Cornstarch | 529.5 | 486.1 | 494.3 | 529.9 | 489.9 |
| Sucrose | 100 | 100 | 100 | 100 | 100 |
| Soybean oil | 70.0 | 52.4 | 69.9 | 69.6 | 52.5 |
| Cellulose | 50 | 0 | 4.6 | 18.7 | 0 |
| Outer layer fraction | - | 176.1 | - | - | - |
| Defatted outer layer fraction | - | - | 138.9 | - | - |
| Outer layer NDF fraction | - | - | - | 32.9 | - |
| Germinates outer layer fraction | - | - | - | - | 169.5 |
| Mineral mixture | 35 | 35 | 35 | 35 | 35 |
| (AlN-93G composition) | | | | | |
| Vitamin mixture | 10 | 10 | 10 | 10 | 10 |
| (AlN-93 composition) | | | | | |
| Choline bitartrate | 2.5 | 2.5 | 2.5 | 2.5 | 2.5 |
| t-Butyfhydroquinone | 0.014 | 0.014 | 0.014 | 0.014 | 0.014 |
| | 1000.00 | 1000.00 | 1000.00 | 1000.00 | 1000.00 |

### (Breeding of experimental animals)

Four-week-old male KK mice (CLEA Japan) were preliminarily bred for 1 week in a breeding room with a temperature of 22 ± 3°C (allowable range: 18 to 26°C), a relative humidity of 55 ± 20% (allowable range: 30 to 80%), a ventilation frequency of 12 times/hr and a light-dark cycle of 12 hours (illumination: 8:00 to 20:00). The general state of each mouse was observed each day during the preliminary feeding period, and the body weight was measured on the day following reception and on the final day of the period.

### (Feeding of diets)

After selecting 24 mice whose general states had been satisfactory during the preliminary feeding period, they were randomly divided into five groups (8 mice per group) so that the means and variances of body weights were roughly equal in the five groups. The mice in each group were allowed free access to the diet and tap water for 2 months.

### (Measurement of fat weights, posterior abdominal wall adipocyte size, serum leptin level and serum insulin level)

Upon completion of the two-month feeding period, the KK mice were dissected and their fat weights, posterior abdominal wall adipocyte size (long diameter), serum leptin level and serum insulin level were measured (fasting for 4 hours before dissection).

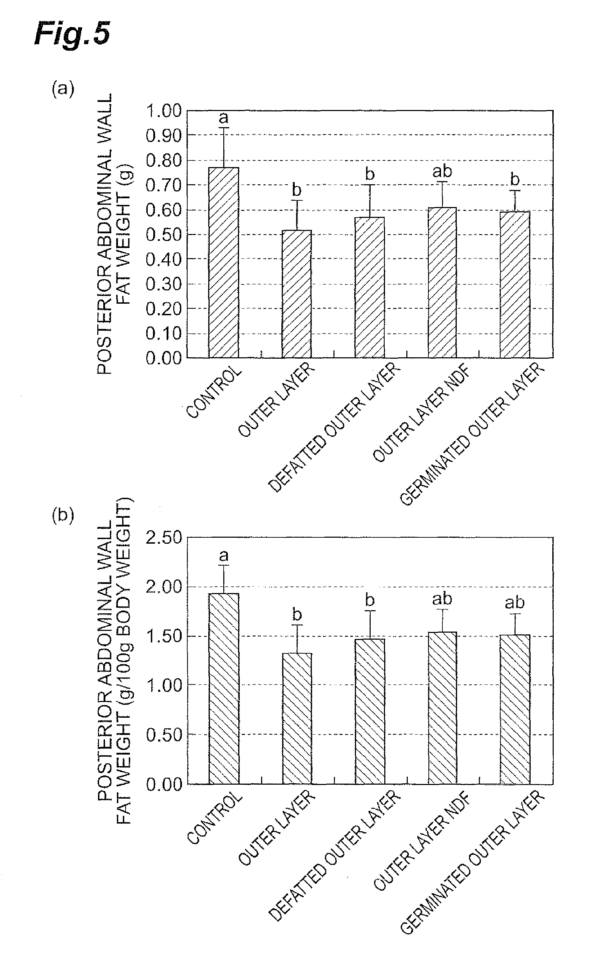

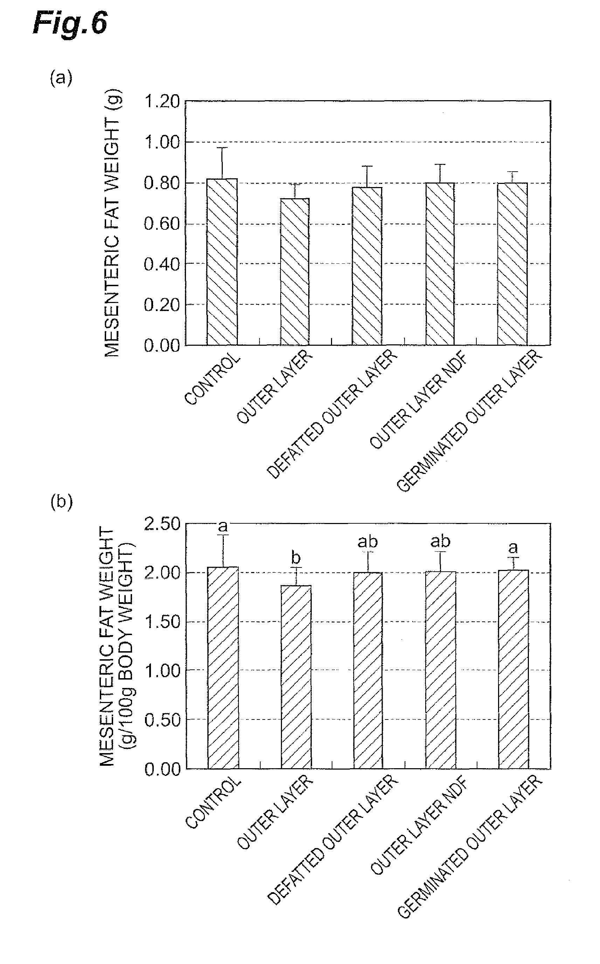

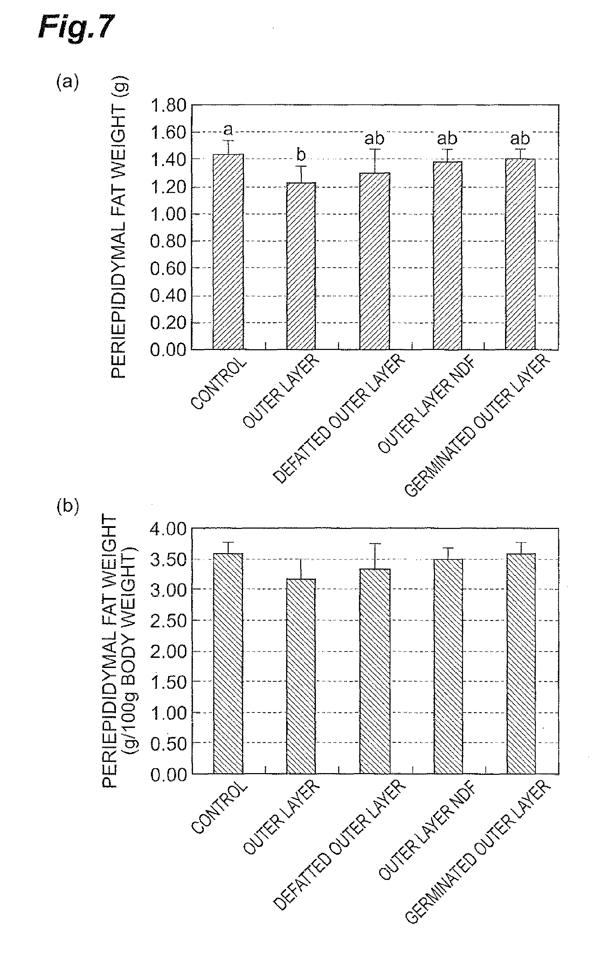

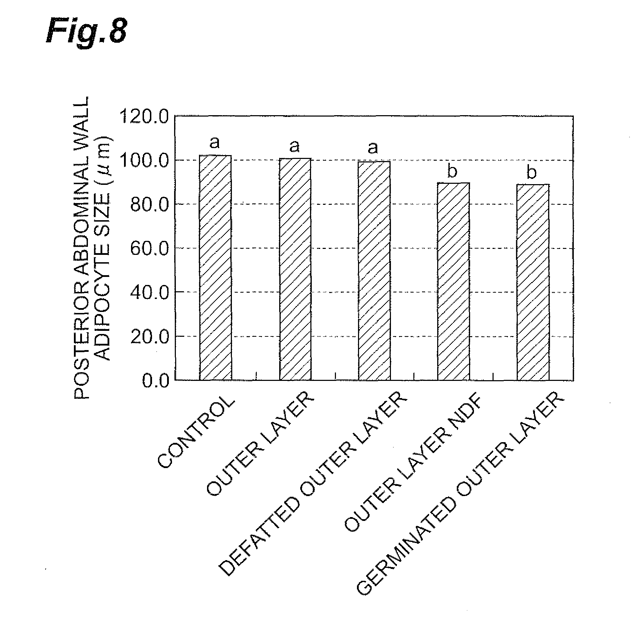

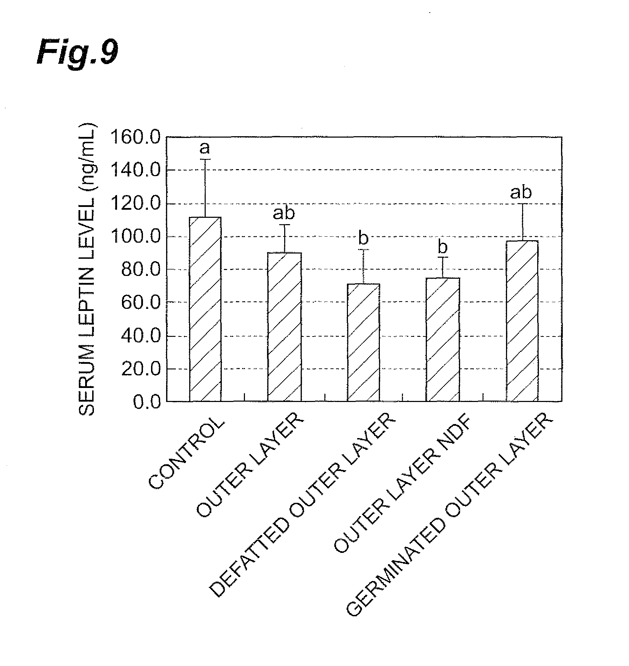

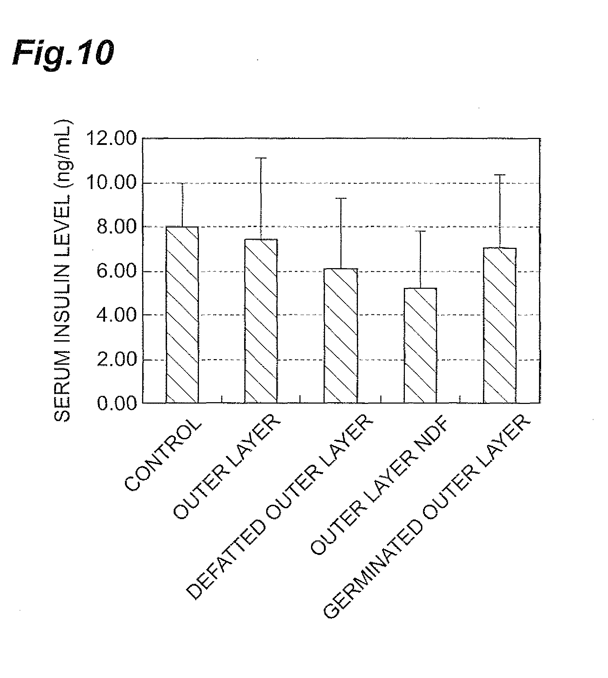

Tables 13 and 14 and Figs. 5 to 7 show the weights of fat that had accumulated around different organs of the KK mice. Table 15 and Fig. 8 show the posterior abdominal wall adipocyte size (long diameter) for the KK mice. Table 16 and in Figs. 9 and 10 show the serum leptin level and serum insulin level of the KK mice. Figs. 5 to 7 are graphs showing the posterior abdominal wall fat weight, mesenteric fat weight and periepididymal fat weight, respectively. In each figure, graph (a) (corresponding to Table 13) shows the total fat weight (g), and graph (b) (corresponding to Table 14) shows the fat weight per 100 g of body weight of the mice (g/100g body weight). Fig. 8 is a graph showing the posterior abdominal wall adipocyte size (long diameter). Figs. 9 and 10 are graphs showing the serum leptin level and serum insulin level, respectively. The data in the table are expressed as mean ± standard deviation. The alphabet superscripts in the tables and graphs represent the results of statistical analysis by the Turkey-Kramer method, and there are statistically significant differences (p < 0.05) between the data with different alphabet superscripts.

**[Table 13]**

| | Posterior abdominal wall fat (g) | Mesenteric fat (g) | Periepididymal fat (g) |
|---|---|---|---|
| Control | 0.77 ± 0.16 ^{a} | 0.82 ± 0.15 | 1.44 ± 0.10 ^{a} |
| Outer layer | 0.52 ± 0.12 ^{b} | 0.72 ± 0.07 | 1.23 ± 0.12 ^{b} |
| Defatted outer layer | 0.57 ± 0.13 ^{b} | 0.78 ± 0.10 | 1.30 ± 0.18 ^{ab} |
| Outer layer NDF | 0.61 ± 0.10 ^{ab} | 0.80 ± 0.09 | 1.38 ± 0.10 ^{ab} |
| Germinated outer layer | 0.59 ± 0.09 ^{b} | 0.80 ± 0.05 | 1.40 ± 0.08 ^{ab} |

**[Table 14]**

| | Posterior abdominal wall fat (g/100g body weight) | Mesenteric fat (g/100g body weight) | Periepididymal fat (g/100g body weight) |
|---|---|---|---|
| Control | 1.92 ± 0.29 ^{a} | 2.05 ± 0.33 ^{a} | 3.58 ± 0.18 |
| Outer layer | 1.32 ± 0.29 ^{b} | 1.86 ± 0.19 ^{b} | 3.16 ± 0.33 |
| Defatted outer layer | 1.46 ± 0.30 ^{b} | 2.00 ± 0.21 ^{ab} | 3.33 ± 0.41 |
| Outer layer NDF | 1.54 ± 0.23 ^{ab} | 2.01 ± 0.20 ^{ab} | 3.48 ± 0.19 |
| Germinated outer layer | 1.51 ± 0.22 ^{ab} | 2.03 ± 0.13 ^{a} | 3.58 ± 0.18 |

**[Table 15]**

| | Posterior abdominal wall adipocyte size (µm) |
|---|---|
| Control | 101.9 ± 3.2^{a} |
| Outer layer | 100.7 ± 2.1 ^{a} |
| Defatted outer layer | 99.6 ± 2.7 ^{a} |
| Outer layer NDF | 89.7 ± 3.2 ^{b} |
| Germinated outer layer | 89.2 ± 2.9 ^{b} |

**[Table 16]**

| | Serum leptin level (ng/mL) | Serum insulin level (ng/mL) |
|---|---|---|
| Control | 111.9 ± 34.4 ^{a} | 8.00 ± 1.94 |
| Outer layer | 89.6 ± 17.6 ^{ab} | 7.43 ± 3.66 |
| Defatted | 70.9 ± 20.6 ^{b} | 6.11 ± 3.15 |
| Outer layer NDF | 74.2 ± 13.4 ^{b} | 5.23 ± 2.59 |
| Germinated outer layer | 96.7 ± 22.8 ^{ab} | 7.07 ± 3.26 |

As shown in Tables 13 and 14 and Figs. 5 to 7, the outer layer fraction-treated group, defatted outer layer fraction-treated group, outer layer NDF fraction-treated group and germinated outer layer fraction-treated group all exhibited lower values of posterior abdominal wall fat weight, mesenteric fat weight and periepididymal fat weight than the control group (except for the periepididymal fat weight in the germinated outer layer fraction-treated group). These results demonstrate that the outer layer (barley bran) fraction, defatted outer layer fraction, outer layer NDF fraction and germinated outer layer fraction have a visceral fat reducing effect.

As shown in Table 15 and Fig. 8, the outer layer fraction-treated group, defatted outer layer fraction-treated group, outer layer NDF fraction-treated group and germinated outer layer fraction-treated group all exhibited lower values of posterior abdominal wall adipocyte size than the control group. In particular, the outer layer NDF fraction-treated group and germinated outer layer fraction-treated group exhibited notably lower values. These results demonstrate that the outer layer (barley bran) fraction, defatted outer layer fraction, outer layer NDF fraction and germinated outer layer fraction have an adipocyte hypertrophy inhibiting effect.

Also, as shown in Table 16 and Figs. 9 and 10, the outer layer fraction-treated group, defatted outer layer fraction-treated group, outer layer NDF fraction-treated group and germinated outer layer fraction-treated group all exhibited lower values of serum leptin level and serum insulin level than the control group. In particular, the defatted outer layer fraction-treated group and outer layer NDF fraction-treated group exhibited notably lower values. These results demonstrate that the outer layer (barley bran) fraction, defatted outer layer fraction, outer layer NDF fraction and germinated outer layer fraction have a blood leptin level lowering effect and blood insulin level lowering effect.

Example 3 demonstrates that barley bran, an amylase-treated product thereof or a water-insoluble component of an amylase-treated product of barley bran has a visceral fat reducing effect, adipocyte hypertrophy inhibiting effect, blood leptin level lowering effect and blood insulin level lowering effect, and is therefore useful as an ameliorative or preventive agent for metabolic syndrome.

## Claims

1. An ameliorative or preventive agent for metabolic syndrome comprising a barley bran or an α-amylase-treated product thereof as an active ingredient.

2. An ameliorative or preventive agent for metabolic syndrome comprising a water-insoluble component of an α-amylase-treated product of a barley bran as an active ingredient.

3. The ameliorative or preventive agent for metabolic syndrome according to claim 2, the agent comprising a cell wall component as the water-insoluble component.

4. The ameliorative or preventive agent for metabolic syndrome according to claim 3, the agent comprising insoluble dietary fiber as the cell wall component.

5. The ameliorative or preventive agent for metabolic syndrome according to claim 4, further comprising protein, lipid and ash,
wherein the protein, lipid and ash contents are 1 to 300 parts by weight, 1 to 100 parts by weight and 1 to 80 parts by weight, respectively, per 100 parts by weight of the insoluble dietary fiber.

6. The ameliorative or preventive agent for metabolic syndrome according to claim 4 or 5, wherein the insoluble dietary fiber is at least one from among hemicellulose, cellulose, lignin, pectin and insoluble β-glucan.

7. The ameliorative or preventive agent for metabolic syndrome according to any one of claims 1 to 6, wherein the barley bran is obtained by milling threshed barley grain to a milling degree of 5% to 30%.

8. The ameliorative or preventive agent for metabolic syndrome according to any one of claims 1 to 7, which has a visceral fat reducing effect.

9. The ameliorative or preventive agent for metabolic syndrome according to any one of claims 1 to 8, which has an adipocyte hypertrophy inhibiting effect.

10. The ameliorative or preventive agent for metabolic syndrome according to any one of claims 1 to 9, which has a blood leptin level lowering effect.

11. The ameliorative or preventive agent for metabolic syndrome according to any one of claims 1 to 10, which has a blood insulin level lowering effect.

12. A beverage containing the ameliorative or preventive agent for metabolic syndrome according to any one of claims 1 to 11.

13. A food containing the ameliorative or preventive agent for metabolic syndrome according to any one of claims 1 to 11.

14. A feed containing the ameliorative or preventive agent for metabolic syndrome according to any one of claims 1 to 11.
